# EUROPEAN PATENT APPLICATION

(11) **EP 3 556 229 A1**
(43) Date of publication of application: **23.10.2019**
(21) Application number: 17880700.4
(22) Date of filing: 15.12.2017
(51) Int. Cl.: A24F 47/00

(54) **ELECTRONIC CIGARETTE AND CONTROL METHOD THEREFOR**

(30) Priority: 15.12.2016 CN 201611163578
(71) Applicant: Changzhou Patent Electronic Technology Co., Ltd, Jiangsu 213022 (CN)
(72) Inventor: QIU, Weihua, Changzhou Jiangsu 213125 (CN)
(74) Representative: Zaboliene, Reda
(86) International application number: PCT/CN2017/116650
(87) International publication number: WO 2018/108172

(57) **Abstract**

Disclosed are an electronic cigarette and a control method therefor. The electronic cigarette comprises: a power source device (5), a cartridge device (1), an atomization device (21), a vapour channel (22), a separating device (23) and a controller (4). The atomization device (21), the separating device (23) and the controller (4) are all electrically connected to the power source device (5). The atomization device (21) is configured to atomize a vapour-forming substance in the cartridge device (1) to generate vapour. The controller (4) is configured to generate a control signal to control the operation of the separating device (23). The separating device (23) is arranged in the vapour channel (22) and is in signal connection with the controller (4). The separating device (23) is configured to carry out, after receiving the control signal from the controller (4), separation treatment on vapour flowing through the separating device (23). Vapour in the vapour channel (22) can be filtered by means of the separating device (23), so that nicotine in the vapour is separated out. The filtered vapour does not contain nicotine, but a user can still inhale the filtered vapour to obtain a feeling of smoking, simulating the feel of smoking.

## Description

The present disclosure claims priority to Chinese patent application No. 201611163578.4, filed on December 15, 2016 and entitled "electronic cigarette and control method therefor", the entire disclosure of which is hereby incorporated by reference.

### TECHNICAL FIELD

The present disclosure relates to the technical field of simulated smoking, and more particularly to an electronic cigarette and a control method therefor.

### BACKGROUND

E-cigarettes, also known as electronic cigarettes, provide a smoking substitute for smokers. An electronic cigarette is a smoking device with no open flames which typically includes an atomizing device powered by a battery for atomizing liquid nicotine when it is activated by a user.

However, nicotine can be addictive or dependent. Repeated use of nicotine also increases heart speed, raises blood pressure and reduces appetite. Large doses of nicotine can cause vomiting and nausea, and in severe cases, death can occur.

### SUMMARY

In view of the problems existed in the prior art, the present disclosure aims to provide an electronic cigarette and a control method therefor, reducing and alleviating the dependence of the central nervous system on nicotine, reducing the harm of nicotine to the human body, and being healthier.

To achieve the above object, the present disclosure provides an electronic cigarette. The electronic cigarette includes a power supply device, a cartridge device, an atomizing device, a smoke passage, a separating device and a controller;
wherein the atomizing device, the separating device and the controller are each electrically connected to the power supply device;
the atomizing device is configured to atomize the smoking-generating substance in the cartridge device to generate smoke;
the controller is configured to generate a control signal to control the operation of the separating device;
the separating device is disposed in the smoke passage and is signally connected to the controller, the separating device is configured to perform a separation treatment on the smoke flowing through the separating device after receiving the control signal from the controller.

Further, the separating device is provided with at least two separation levels, two adjacent separation levels gradually increase or gradually decrease the degree of separation.

Further, the separating device includes a centrifuge, the centrifuge is configured to centrifugally separate the smoke in the smoke passage so that nicotine in the smoke is centrifugally separated out.

Further, the separating device further includes a collection device connected to the centrifuge, the collection device is configured for collecting the nicotine centrifugally separated out by the centrifuge.

Further, the separating device is provided with a substance which chemically reacts with nicotine so that nicotine is separated out from the nicotine carrier.

Further, the collection device is signally connected to the controller, and after the collection amount of nicotine in the collection device reaches a predetermined value, the controller issues a reminder signal for replacing the separating device.

Further, the electronic cigarette further includes a detecting device or an input device for acquiring the concentration of nicotine content, and the controller acquires the concentration information of nicotine content detected by the detecting device or inputted through the input device.

To achieve the above object, the present disclosure also provides a method for controlling an electronic cigarette, the method includes steps:
controlling the atomizing device to start work, the atomizing device being configured to atomize a smoke-generating substance in the cartridge device to generate smoke;
determining whether the amount of nicotine inhaled exceeds a preset value;
controlling the separating device to start work if the amount of nicotine inhaled exceeds the preset value;
wherein the separating device is disposed in the smoke passage and is signally connected with the controller, the separating device is configured to perform a separation treatment on the smoke flowing through the separating device after receiving a control signal from the controller.

Further, the method further includes steps:
calculating the amount of smoke inhaled; and
calculating the amount of nicotine inhaled according to the amount of smoke inhaled.

Further, the step of calculating the amount of smoke inhaled includes: calculating the amount of smoke inhaled according to the suction time, the voltage parameter and the number of puffs; or calculating the amount of smoke inhaled according to the suction time, the power parameter and the number of puffs.

Further, the amount of nicotine inhaled is the product of the amount of smoke inhaled and the concentration of nicotine content.

Further, the preset value is a nicotine inhaling threshold in a preset time period or is a nicotine inhaling threshold in a single puff.

Further, the method further includes: determining a difference between the amount of nicotine inhaled and the preset value, and controlling, according to the difference, the separating device to select a separation level for separating the smoke.

The electronic cigarette and the control method therefor provided by the embodiments of the present disclosure can determine whether the amount of nicotine inhaled by the user exceeds a preset value during smoking. If the amount of nicotine inhaled by the user exceeds the preset value, the separating device is activated to start work, the separating device filters the smoke in the smoke passage, so that nicotine in the smoke is separated out, the filtered smoke does not contain nicotine, and the user can still inhale the filtered smoke to achieve the feeling of simulated smoking.

### BRIEF DESCRIPTION OF THE DRAWINGS

FIG. 1 is a schematic structural view of an electronic cigarette according to a first embodiment of the present disclosure;
FIG. 2 is a flow chart of a method for controlling an electronic cigarette according to a second embodiment of the present disclosure;
FIG. 3 is a flow chart of a method for controlling an electronic cigarette according to a third embodiment of the present disclosure.

The present disclosure will be further illustrated by the following detailed description in conjunction with the accompanying drawings.

### DETAILED DESCRIPTION OF PREFERRED EMBODIMENTS

The present disclosure will be described in detail below in conjunction with the embodiments shown in the drawings. However, the embodiments do not limit the present disclosure, and structural or functional changes made by those skilled in the art based on these embodiments are included in the scope of the present disclosure.

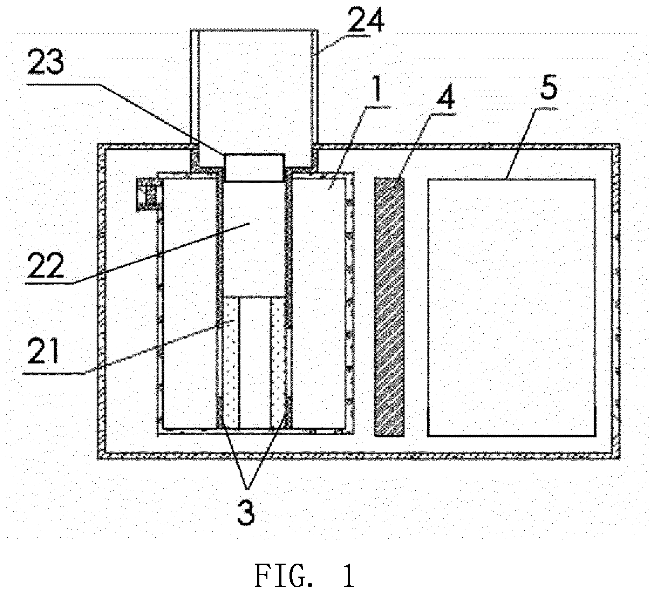

FIG. 1 is a schematic structural view of an electronic cigarette according to a first embodiment of the present disclosure. The electronic cigarette includes a power supply device 5, a cartridge device 1, an atomizing device 21, a smoke passage 22, a separating device 23, and a controller 4.

The atomizing device 21, the separating device 23, and the controller 4 are each electrically connected to the power supply device 5.

The cartridge device 1 is configured to store a smoke-generating substance. The smoke-generating substance may specifically be nicotine-containing cigarette liquid or solid cigarette paste or tobacco, or the like. In one embodiment, the cartridge device 1 can be detached freely, and can be covered with a sealing cover after being detached for storing the smoke-generating substance for a long time, in order to prevent oxidation or mildew reaction of the smoke-generating substance.

The atomizing device 21 is configured to atomize the smoke-generating substance in the cartridge device 1 to generate smoke, and the smoke passes through the smoke passage 22 for the user to inhale. For example, a mouthpiece may be provided for being sucked by the mouth of a user to inhale the smoke of the electronic cigarette. There may be various examples of the atomizing device 21, and a relatively common example is that a metal heating wire is wound around a liquid guiding member such as cotton, cotton cloth or porous ceramic, or the metal heating wire is wound by the liquid guiding member. In addition to the metal heating wire, the heating element can also be a heating piece, a heating rod, or the like.

In this embodiment, the atomizing device 21 absorbs the cigarette liquid from the cartridge device 1 by capillary action to atomize the cigarette liquid and generate smoke. In one embodiment, the capillary action may be provided by cotton, cotton cloth, porous ceramic, metal foam, or the like.

The separating device 23 is disposed in the smoke passage 22 for physically or chemically separating the smoke of the smoke passage 22 so that nicotine in the smoke is separated out. In one embodiment not shown, the separating device 23 is provided with at least two separation levels, such as low separation level and high separation level, depending on the degree of separation. When the amount of smoke or the amount of nicotine inhaled by the user is far from a preset value, the separating device 23 is turned on the low separation level at this time to separate out a small portion of the nicotine from the generated smoke. When the amount of smoke or the amount of nicotine inhaled by the user is almost the same as or has reached the preset value, the separating device 23 is turned on the high separation level at this time to separate out most or all of the nicotine from the generated smoke. The separation levels of the separating device 23 can be set according to the actual needs, which can also include three, four or five separation levels. It can be understood that the more the separation levels are set, the finer the control of the separation effect is.

With regard to physical separation, in one example of this embodiment, the separating device may include a centrifuge and a collection device (not shown).

The centrifuge is configured to centrifugally separate the smoke in the smoke passage, so that nicotine in the smoke is separated out from the nicotine carrier by the centrifugal force. The centrifuge can be a micro centrifuge, for example, a DC4010 centrifugal fan, the rotation speed can reach 7500-9000r/min, and the volume is relatively small with a size only 20^{∗}20^{∗}6mm, which is enough to install in the smoke passage.

According to one example of this embodiment, the electronic cigarette further includes a collection device for collecting the nicotine centrifugally separated out by the centrifuge. When the nicotine collected by the collection device reaches a certain liquid level or a certain weight, the user may be reminded to replace the separating device, or replace the collection device, or pour out the nicotine in the collection device.

With regard to chemical separation, in one example of this embodiment, the separating device is provided with a chemical substance capable of chemically reacting with nicotine, for example, an acid substance which can be neutralized with nicotine. When the separating device is activated, the smoke flows through the separating device, and the nicotine carried by the nicotine carrier reacts chemically with the chemical substance in the separating device. For example, when the chemical substance is an acid substance, the nicotine is an alkaloid, so the two react with each other to form nicotine salts.

The controller 4 is configured to control the atomizing device 21 and the separating device 23 to start or stop working.

In one embodiment, the electronic cigarette may further include an input device or an airflow sensor.

The controller 4 acquires the signals inputted by the input device or the airflow signals detected by the airflow sensor, and controls the operation of the atomizing device 21 according to the signals inputted by the input device or the airflow signals detected by the airflow sensor.

The input device can be disposed on the outer casing of the electronic cigarette, including but not limited to a keyboard and a touch screen. The user can enter his or her own preferences through the input device such as a keyboard or a touch screen, and can send his or her own smoking instructions to cause the atomizing device 21 to start work.

If the electronic cigarette includes the airflow sensor, when the user sucks the electronic cigarette, an airflow is generated, the airflow sensor can detect the airflow and generate an airflow signal, and when the controller 4 acquires the airflow signal detected by the airflow sensor, the atomizing device 21 is controlled to start work.

In this embodiment, the controller 4 controls the separating device 23 to start work, including:

Calculating the amount of smoke inhaled by the user, wherein calculating the amount of smoke inhaled by the user includes: calculating the amount of smoke inhaled by the user according to the user's suction time, the voltage parameter and the number of puffs; or calculating the amount of smoke inhaled by the user according to the user's suction time, the power parameter and the number of puffs. As an example, if the user's suction time is t, the power parameter is P and the number of puffs is n, then the amount of smoke S inhaled by the user may be calculated according to the following formula: S=k^{∗}n^{∗}P^{∗}t, wherein k is the coefficient between the amount of smoke and the power. As another example, if the user's suction time is t, the voltage parameter is V and the number of puffs is n, then the amount of smoke S inhaled by the user may be calculated according to the following formula: S=k^{∗}n^{∗}V^{∗}t, wherein k is the coefficient between the amount of smoke and the voltage.

Calculating the amount of nicotine inhaled by the user according to the amount of smoke inhaled by the user, wherein the amount of nicotine inhaled by the user is the product of the amount of smoke inhaled by the user and the concentration of nicotine content. As an example, the concentration of nicotine content is a, and the concentration a of nicotine content may be obtained through the following methods. 1) The electronic cigarette is provided with the aforementioned input device, the input device may be disposed on the outer casing of the electronic cigarette, including but not limited to a keyboard and a touch screen. The input device can input the value of the concentration a of nicotine content, the controller 4 can obtain the value of the concentration a of nicotine content inputted by the input device and store it. 2) The electronic cigarette is provided with a detecting device for detecting the concentration of nicotine content, and the detecting device can be set in the cartridge device 1 or in the smoke passage 22. The concentration of nicotine content in the smoking-generating substance can be detected by the detecting device, the controller can obtain the concentration of nicotine content in the smoking-generating substance detected by the detecting device and store it. The amount of nicotine inhaled by the user M = the amount of smoke S inhaled by the user * the concentration a of nicotine content, that is, the product of the amount of smoke inhaled by the user and the concentration of nicotine content.

Determining whether the amount of nicotine inhaled by the user exceeds a preset value. In this embodiment, the preset value may be a nicotine inhaling threshold in a preset time period. As an example, the preset value may be a daily, weekly, or monthly nicotine inhaling upper limit desired by the user, or may be a recommended value of nicotine inhaling, and the recommended value may be pre-stored in the controller 4.

Controlling, through the controller 4, the separating device 23 to start work if the amount of nicotine inhaled by the user exceeds the preset value.

The electronic cigarette provided by the embodiment of the present disclosure can determine whether the amount of nicotine inhaled by the user exceeds a preset value during smoking. If the amount of nicotine inhaled by the user exceeds the preset value, the separating device is activated to start work, the separating device filters the smoke in the smoke passage, so that nicotine in the smoke is separated out, the filtered smoke does not contain nicotine, but the user can still inhale the filtered smoke to achieve the feeling of simulated smoking, to relieve the dependence of the central nervous system on nicotine and reduce the harm of nicotine to the human body.

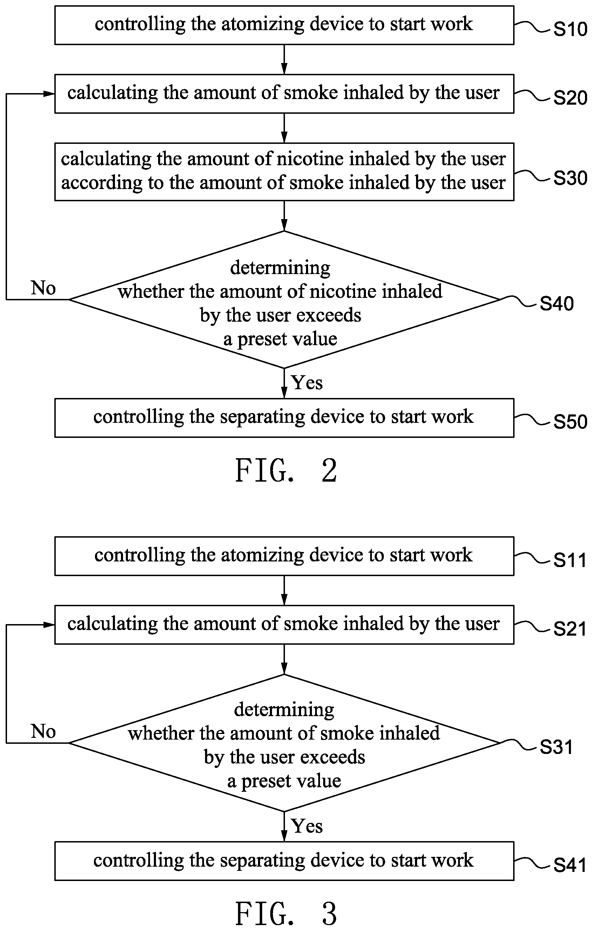

Referring to FIG. 2, FIG. 2 is a method for controlling an electronic cigarette according to a second embodiment of the present disclosure. The method includes the following steps:
S10: controlling the atomizing device to start work.

Specifically, the atomizing device is configured to atomize the smoke-generating substance in the cartridge device to generate smoke.

S20: calculating the amount of smoke inhaled by the user.

Calculating the amount of smoke inhaled by the user includes: calculating the amount of smoke inhaled by the user according to the user's suction time, the voltage parameter and the number of puffs; or calculating the amount of smoke inhaled by the user according to the user's suction time, the power parameter and the number of puffs. As an example, if the user's suction time is t, the power parameter is P and the number of puffs is n, then the amount of smoke S inhaled by the user may be calculated according to the following formula: S=k^{∗}n^{∗}P^{∗}t, wherein k is the coefficient between the amount of smoke and the power. As another example, if the user's suction time is t, the voltage parameter is V and the number of puffs is n, then the amount of smoke S inhaled by the user may be calculated according to the following formula: S=k^{∗}n^{∗}V^{∗}t, wherein k is the coefficient between the amount of smoke and the voltage.

S30: calculating the amount of nicotine inhaled by the user according to the amount of smoke inhaled by the user.

The amount of nicotine inhaled by the user is the product of the amount of smoke inhaled by the user and the concentration of nicotine content. As an example, the concentration of nicotine content is a, and the concentration a of nicotine content may be obtained through the following methods. 1) The electronic cigarette is provided with the aforementioned input device, the input device may be disposed on the outer casing of the electronic cigarette, including but not limited to a keyboard and a touch screen. The input device can input the value of the concentration a of nicotine content, the controller 4 can obtain the value of the concentration a of nicotine content inputted by the input device and store it. 2) The electronic cigarette is provided with a detecting device for detecting the concentration of nicotine content, and the detecting device can be set in the cartridge device 1 or in the smoke passage 22. The concentration of nicotine content in the smoking-generating substance can be detected by the detecting device, the controller can obtain the concentration of nicotine content in the smoking-generating substance detected by the detecting device and store it. The amount of nicotine inhaled by the user M = the amount of smoke S inhaled by the user * the concentration a of nicotine content, that is, the product of the amount of smoke inhaled by the user and the concentration of nicotine content.

It can be understood that steps S20 and S30 are not necessarily to be executed by the electronic cigarette. In this embodiment, steps S20 and S30 can, of course, be executed by the controller of the electronic cigarette. In another embodiment not shown, step S20 or S30 can also be executed by an external device such as a mobile phone or a mobile tablet PC connected to the electronic cigarette through data transmission connection such as a Bluetooth connection, an infrared connection or a WiFi connection. In addition, steps S20 and S30 can also be executed separately, for example, step S20 is executed by the electronic cigarette, while step S30 is executed by an external device connected to the electronic cigarette through data transmission connection.

S40: determining whether the amount of nicotine inhaled by the user exceeds a preset value.

In this embodiment, the preset value may be a nicotine inhaling threshold in a preset time period. As an example, the preset value may be a daily, weekly, or monthly nicotine inhaling upper limit desired by the user, or may be a recommended value of nicotine inhaling, and the recommended value may be pre-stored in the controller 4.

It can be understood that the method of this embodiment may further include: determining a difference between the amount of nicotine inhaled by the user and the preset value, and controlling, according to the difference, the separating device to select a separation level for separating the smoke. For example, when the amount of nicotine inhaled by the user is closer to the preset value, that is, the difference is small, the separating device is controlled to select the high separation level to separate nicotine out from the smoke, so that nicotine in the smoke is separated out to a greater extent. When it is determined that the difference between the amount of nicotine inhaled by the user and the preset value is large, that is, the amount of nicotine inhaled by the user is far from the preset value, the separating device is controlled to select the low separation level to separate nicotine out from the smoke, so that nicotine in the smoke is not separated or is separated to a lesser extent.

S50: controlling the separating device to start work if the amount of nicotine inhaled by the user exceeds the preset value.

Specifically, the separating device is disposed in the smoke passage and is signally connected to the controller, and the separating device is configured to perform a separation treatment on the smoke flowing through the separating device after receiving the control signal from the controller, so that nicotine in the smoke is separated out.

It can be understood that, with reference to FIG. 3, a third embodiment of the present disclosure further provides a method for controlling an electronic cigarette, including the following steps:
S11: controlling the atomizing device to start work.

Specifically, the atomizing device is configured to atomize the smoke-generating substance in the cartridge device to generate smoke.

S21: calculating the amount of smoke inhaled by the user.

Calculating the amount of smoke inhaled by the user includes: calculating the amount of smoke inhaled by the user according to the user's suction time, the voltage parameter and the number of puffs; or calculating the amount of smoke inhaled by the user according to the user's suction time, the power parameter and the number of puffs. As an example, if the user's suction time is t, the power parameter is P and the number of puffs is n, then the amount of smoke S inhaled by the user may be calculated according to the following formula: S=k^{∗}n^{∗}P^{∗}t, wherein k is the coefficient between the amount of smoke and the power. As another example, if the user's suction time is t, the voltage parameter is V and the number of puffs is n, then the amount of smoke S inhaled by the user may be calculated according to the following formula: S=k^{∗}n^{∗}V^{∗}t, wherein k is the coefficient between the amount of smoke and the voltage.

It can be understood that step S21 is not necessarily to be executed by the electronic cigarette. In this embodiment, step S21 can, of course, be executed by the controller of the electronic cigarette. In another embodiment not shown, step S21 can also be executed by an external device such as a mobile phone or a mobile tablet PC connected to the electronic cigarette through data transmission connection such as a Bluetooth connection, an infrared connection or a WiFi connection.

S31: determining whether the amount of smoke inhaled by the user exceeds a preset value.

In this embodiment, the preset value may be a smoke inhaling threshold in a preset time period. As an example, the preset value may be a daily, weekly, or monthly smoke inhaling upper limit desired by the user, or may be a recommended value of smoke inhaling, and the recommended value may be pre-stored in the controller 4.

S41: controlling the separating device to start work if the amount of smoke inhaled by the user exceeds the preset value.

Compared with the second embodiment, the method for controlling the electronic cigarette provided by this embodiment can estimate or determine nicotine inhaling level of the user by judging the amount of inhaled smoke, and does not need to further accurately calculate the nicotine inhaling. Specifically, the method in this embodiment is to determine whether the amount of smoke inhaled by the user exceeds the preset value during smoking, and if the amount of smoke inhaled exceeds the preset value, the separating device is activated to start work, and the separating device filters the smoke in the smoke passage to separate nicotine out from the smoke, the filtered smoke does not contain nicotine, but the user can still inhale the filtered smoke to achieve the feeling of simulated smoking, to reduce and relieve the dependence of the central nervous system on nicotine, and reduce the harm of nicotine to the human body.

It can be understood that the method of this embodiment may further include: determining a difference between the amount of nicotine inhaled by the user and the preset value, and controlling, according to the difference, the separating device to select a separation level for separating the smoke. For example, when the amount of nicotine inhaled by the user is closer to the preset value, that is, the difference is small, the separating device is controlled to select the high separation level to separate nicotine out from the smoke, so that nicotine in the smoke is separated out to a greater extent. When it is determined that the difference between the amount of nicotine inhaled by the user and the preset value is large, that is, the amount of nicotine inhaled by the user is far from the preset value, the separating device is controlled to select the low separation level to separate nicotine out from the smoke, so that nicotine in the smoke is not separated or is separated to a lesser extent.

It should be understood that although the present disclosure is described in terms of the embodiments, but not each of the embodiments contains only an independent technical solution, and such description is only for the sake of clarity. Technicians in the art should take the specification as a whole, and the technical solutions in the embodiments can also be appropriately combined to form other embodiments understandable by those skilled in the art.

The detailed descriptions set forth above are merely illustrative of the possible embodiments of the present disclosure, and are not intended to limit the scope of the present disclosure. Variations and modifications to the above embodiments within the spirit of the present disclosure are intended to fall within the scope of the present disclosure.

## Claims

1. An electronic cigarette, comprising a power supply device, a cartridge device, an atomizing device, a smoke passage, a separating device and a controller;
wherein the atomizing device, the separating device and the controller are each electrically connected to the power supply device;
the atomizing device is configured to atomize the smoking-generating substance in the cartridge device to generate smoke;
the controller is configured to generate a control signal to control the operation of the separating device;
the separating device is disposed in the smoke passage and is signally connected to the controller, the separating device is configured to perform a separation treatment on the smoke flowing through the separating device after receiving the control signal from the controller.

2. The electronic cigarette according to claim **1,** wherein the separating device is provided with at least two separation levels, two adjacent separation levels gradually increase or gradually decrease the degree of separation.

3. The electronic cigarette according to claim **1,** wherein the separating device comprises a centrifuge, the centrifuge is configured to centrifugally separate the smoke in the smoke passage so that nicotine in the smoke is centrifugally separated out.

4. The electronic cigarette according to claim **3,** wherein the separating device further comprises a collection device connected to the centrifuge, the collection device is configured for collecting the nicotine centrifugally separated out by the centrifuge.

5. The electronic cigarette according to claim **1,** wherein the separating device is provided with a substance which chemically reacts with nicotine so that nicotine is separated out from the nicotine carrier.

6. The electronic cigarette according to claim **4,** wherein the collection device is signally connected to the controller, and after the collection amount of nicotine in the collection device reaches a predetermined value, the controller issues a reminder signal for replacing the separating device.

7. The electronic cigarette according to claim **1,** wherein the electronic cigarette further comprises a detecting device or an input device for acquiring the concentration of nicotine content, and the controller acquires the concentration information of nicotine content detected by the detecting device or inputted through the input device.

8. A method for controlling an electronic cigarette, the method comprising steps:
controlling the atomizing device to start work, the atomizing device being configured to atomize a smoke-generating substance in the cartridge device to generate smoke;
determining whether the amount of nicotine inhaled exceeds a preset value;
controlling the separating device to perform a separation treatment on the smoke flowing through the separating device if the amount of nicotine inhaled exceeds the preset value;
wherein the separating device is disposed in the smoke passage and is signally connected with the controller.

9. The method for controlling an electronic cigarette according to claim **8,** wherein the method further comprises steps:
calculating the amount of smoke inhaled; and
calculating the amount of nicotine inhaled according to the amount of smoke inhaled.

10. The method for controlling an electronic cigarette according to claim **9,** wherein the step of calculating the amount of smoke inhaled comprises: calculating the amount of smoke inhaled according to the suction time, the voltage parameter and the number of puffs; or calculating the amount of smoke inhaled according to the suction time, the power parameter and the number of puffs.

11. The method for controlling an electronic cigarette according to claim **8,** wherein the amount of nicotine inhaled is the product of the amount of smoke inhaled and the concentration of nicotine content.

12. The method for controlling an electronic cigarette according to claim **8,** wherein the preset value is a nicotine inhaling threshold in a preset time period or is a nicotine inhaling threshold in a single puff.

13. The method for controlling an electronic cigarette according to claim **8,** wherein the method further comprises: determining a difference between the amount of nicotine inhaled and the preset value, and controlling, according to the difference, the separating device to select a separation level for separating the smoke.
